# EUROPÄISCHE PATENTSCHRIFT

(11) **EP 0 656 777 B1**
(45) Veröffentlichungstag und Bekanntmachung des Hinweises auf die Patenterteilung: **29.01.1997**
(21) Anmeldenummer: 93919210.0
(22) Anmeldetag: 28.08.1993
(51) Int. Cl.: A61K 31/41

(54) **SPRITZFERTIGE AZOSEMID-INJEKTIONSLÖSUNGEN**
READY-TO-INJECT AZOSEMIDE SOLUTIONS
SOLUTIONS D'AZOSEMIDE A INJECTER, PRETES A L'EMPLOI

(30) Priorität: 31.08.1992 DE 4228926
(43) Veröffentlichungstag der Anmeldung: 14.06.1995
(73) Patentinhaber: BOEHRINGER MANNHEIM GMBH, 68298 Mannheim (DE)
(72) Erfinder: WOOG, Heinrich, D-69514 Laudenbach (DE); GRUBER, Werner, D-69488 Birkenau (DE)
(86) Internationale Anmeldenummer: EP9302331
(87) Internationale Veröffentlichungsnummer: WO9405286

(56) Entgegenhaltungen:
- DE-A- 1 815 922
- DE-A- 2 423 550
- DE-A- 2 556 001

## Beschreibung

Die vorliegende Erfindung betrifft spritzfertige, wässrige Injektionslösungen, die ein organisches Lösungsmittel und als Wirkstoff mindestens ein wasserlösliches, physiologisch gut verträgliches Salz des Azosemids enthalten und ein Verfahren zur Herstellung solcher Injektionslösungen.

Azosemid (2-Chlor-5-(1H-Tetrazol-5-yl)-N⁴-2-thenylsulfanilamid) und dessen wasserlösliche und physiologisch gut vertragliche Salze finden als Diuretikum in der Medizin Anwendung. Vorteilhafterweise läßt sich Azosemid auch in Kombination mit anderen Diuretika oder mit Betablockern verwenden. Solche Kombinationen sind beispielsweise in dem Patent DE 25 56 001 beschrieben.

In DE 25 56 001 wird das Problem gelöst, daß wässrige Lösungen, welche ein Canrenoat oder Kombinationen eines solchen Salzes mit anderen Diuretica enthalten, nicht stabil sind, weil nach der üblichen Hitzesterilisation bei 120° C unerklärliche Trübungen auftreten. Wie in DE 25 56 001 beschrieben wird, war das Instabilitätsproblem zuvor umgangen worden, indem ein Lyophilisat des Canrenoats oder eines dieses enthaltenden Kombinationspräparat durch Auflösen der Trockensubstanz in einem wässrigen Lösungsmittel hergestellt worden war. Diese Zubereitung einer Injektionslösung weist jedoch Nachteile auf: Die Herstellung eines Lyophilsates erfordert einen hohen technischen Aufwand. Die Lyophilisate müssen durch umständliche Sterilfiltration entkeimt werden, wobei sich eine Quote an unsterilen, pyrogenhaltigen Chargen nicht vermeiden läßt. Zudem ist neben der tatsächlich erforderlichen Lösungsmittelampulle auch ein weiteres Handling zum Auflösen des Lyophilisates erforderlich.

Gemäß DE 25 56 001 wird eine bessere Stabilität erreicht, indem der pH-Wert der Lösungen von maximal 10,2 auf einen Wert im Bereich zwischen 10,2 und 11,2 angehoben wird. Die so stabilisierten Injektionslösungen hatten zwar eine brauchbare Venenverträglichkeit, aber ihr hoher pH-Wert ist physiologisch nicht unbedenklich.

Die Venenverträglichkeit einer Injektionslösung hängt im wesentlichen von folgenden Faktoren ab: pH-Wert. Pufferkapazität und Titrationsbasizität bzw. Titrationsacidität.

Die Pufferkapazität wird allgemein definiert als diejenige äquivalente Menge (val an Säure oder Lauge), die erforderlich ist, um den pH-Wert einer Lösung mit dem Volumen von einem Liter um eine pH-Einheit zu verändern. Falls einbasische Säuren oder Basen verwendet werden, entspricht die Angabe val/l der verwendeten Säure oder Base der molaren Menge Mol/l dieser Säure. Da im vorliegenden Fall die verwendeten Lösungen bevorzugt einen pH-Wert im alkalischen Bereich aufweisen, kann alternativ die Pufferkapazität auch als diejenige Menge einer beispielsweise 0,1 normalen HCl-Lösung definiert werden, die benötigt wird, um den pH-Wert einer Lösung von 1 l um eine pH-Einheit abzusenken. Die Bestimmung der Pufferkapazität in Arzneimitteln erfolgt ausgehend von den fertig applizierbaren Infusionslösungen, die neben dem Wirkstoff die sonst in der pharmazeutischen Praxis verwendeten Hilfs- oder Zusatzstoffe enthalten.

Die Titrationsbasizität wird allgemein definiert als diejenige Menge an Säure, die erforderlich ist, um den pH-Wert einer Lösung mit dem Volumen von einem Liter auf den pH-Wert des Blutes (7,2 - 7,4) einzustellen. Im vorliegenden Fall kann die Titrationsbasizität alternativ auch als diejenige Menge einer beispielsweise 0,1 normalen HCl-Lösung definiert werden, die erforderlich ist, um den pH-Wert von 1 l einer Lösung auf den des Blutes abzusenken. Die Titrationsbasizität läßt sich beeinflussen durch eine Änderung des pH-Werts und eine entsprechende Auswahl des Puffers.

Injektionslösungen, deren pH-Wert im Bereich des pH-Wertes des Blutes, also zwischen 7,2 und 7,4 liegen, oder die zwar einen höheren pH-Wert aber eine geringe Pufferkapazität und eine geringe Titrationsbasizität aufweisen, sind sehr gut venenverträglich. Durch die Anhebung des pH-Werts wird bei den in DE 25 56 001 beanspruchten Lösungen u.a. die Löslichkeit des Wirkstoffs erhöht.

Die Löslichkeit - beispielsweise - des Natriumsalzes des Azosemids in Wasser ist - insbesondere bei höheren pH-Werten - an sich gut (165 mg / 100 ml bei pH 8,0 und 201 mg/100 ml bei pH 10,0). Aber klare, stabile Injektionslösungen ohne Rekristallisate in reinem, wäßrigem Medium konnten selbst dann nicht hergestellt werden, wenn ihre Konzentration an Azosemidsalz unter der Sättigungskonzentration lag. Es kommt hinzu, daß spritzfertige, stabile, klare und gut venenverträgliche Azosemid-Injektionslösungen wünschenswert sind, deren Wirkstoffkonzentration wesentlich höher liegt als die oben angegebenen Sättigungslöslichkeiten des Natriumsalzes, nämlich im Bereich zwischen 2 bis 20 mg/1 ml Lösung.

Lösungen mit einer Azosemid-Wirkstoffkonzentration, welche allerdings höchstens am unteren Rand des wünschenswerten Bereichs, d.h. bei 2mg/ml Lösung, liegt, lassen sich mit dem oben erwähnten Verfahren herstellen, bei dem als Zwischenprodukt ein Lyophilisat erzeugt wird, wobei der Wirkstoff in alkalischer Lösung lyophilisiert wird und das Lyophilisat dar in Wasser aufgelöst wird. Die Venenverträglichkeit der so hergestellten Lösungen sind auch bei pH-Werten von 10 gut. Jedoch ist die Partikelanzahl der so hergestellten Lösungen relativ hoch, so daß sie oft die in der Europäischen Pharmakopoe definierten Kritenien nicht erfüllen. Partikel in den aus Lyophilisaten hergestellten Lösungen sind z.B. Gummipartikel, welche von - bei der Herstellung des Lyophilisats eingesetzten - Gummistopfen stammen, oder Teilchen aus Verkrustungen, die sich bei der Lyophilisation bilden und sich nur schwer vollständig lösen lassen.

In der Europäischen Pharmakopoe wird für Injektionslösungen gefordert, daß sie praktisch frei von Schwebeteilchen sind. In der USP XXII (US-Pharmakopoe = amerikanisches Arzneibuch) wie auch in der BP (Britische Pharmakopoe) sind Grenzen für die sichtbaren und nicht sichtbaren Partikel sowie Methoden zur Partikelbestimmung festgeschrieben (die Partikelzahlen/Behältnis dürfen bei Teilchen-Durchmessern von >= 10 µm max. 10000 und bei Durchmessern von >= 25 µm max. 1000 sein).

Hinzu kommen die oben angesprochenen inhärenten Nachteile des Lyophilisations-Verfahrens und daß bei akzeptablen pH-Werten keine Konzentrationen erzielbar sind, welche wesentlich über der unteren Grenze der brauchbaren Dosen liegt.

Aus der DE-OS-24 23 550 und aus dem Artikel "Renal Actions of Azosemide" von J. Greven und O. Heidenreich, veröffentlicht in Arzneimittel-Forschung/Drug Res. 31, (I), Nr. 2 (1981), Seiten 346-350, ist außerdem bekannt, wässrigen Lösungen von Alkalimetall-Azosemidsalzen organische Lösungsmittel zuzusetzen. Versuche haben gezeigt (siehe dazu Tabelle 1 in der Beschreibung), daß sich durch Zugabe solcher Lösungsmittel die Löslichkeit der Alkalimetall-Azosemidsalze erhöhen läßt. Dies ist erstaunlich, da es sich um Salze handelt, deren lipophiler Charakter viel schwächer ausgeprägt ist als der der dazu gehörenden Säuren. Allerdings ist die erzielbare Erhöhung der Löslichkeit nicht so beachtlich, daß sich Lösungen erzeugen lassen, die eine sehr gute Stabilität und gleichzeitig die gewünschte hohe Wirkstoffkonzentration aufweisen.

Es stellt sich deshalb die Aufgabe, relativ hoch konzentrierte Azosemid-Präparationen, die in Lösungen stabil und bei der Injektion auch in höherer Konzentration physiologisch gut verträglich sind, und ein Verfahren zur Herstellung solcher Lösungen anzugeben. Diese Aufgabe wird gemäß den Merkmalen des Patentanspruchs 1 und mit einem Verfahren gemäß den Merkmalen des Anspruchs 8 gelöst.

Zur Stabilisierung während der Sterilisation muß die Injektionslösung gepuffert sein, um einen zu starken Abfall des pH-Werts zu verhindern. Die Salze des Azosemids sind in der Regel Salze einer schwachen Säure und einer starken Base und weisen somit selbst Puffercharakter auf Nur weg die Pufferwirkung der Azosemidsalze zu gering ist, muß zusätzlich Puffer der Lösung zugesetzt werden. Bei der Pufferung wird ein Kompromiß zwischen einer ausreichenden Stabilisierung und einer möglichst geringen Pufferkapazität der Injektionslösung gesucht. Es ist ein günstiger Umstand bei den erfindungsgemäßen Injektionslösungen, daß für ihre Stabilisierung keine starke Pufferung erforderlich ist, so daß die vorteilhaft geringe Pufferkapazität der erfindungsgemäßen Lösungen erhalten werden kann. Die erfindungsgemäßen Lösungen überstehen eine 20 Min. dauernde Sterilisation bei 121° C ohne nennenswerte Partikelbildung und Zersetzung. Wegen der geringen Pufferkapazität führen die erfindungsgemäßen Lösungen nicht zu signifikanten pH-Wert-Veränderungen an der Injektionsstelle, so daß eine unverdünnte Applikation möglich ist.

Die Lösungen können - nach den bisherigen Erfahrungen - in völlig unerwarteter Weise mindestens drei Jahre lang bei Raumtemperatur gelagert werden, ohne daß Trübungen auftreten oder der Wirkstoff sich chemisch verändert. Auch sind diese Injektionslösungen trotz der hohen Wirkstoftkonzentration und des hohen Gehaltes an organischen Lösungsmitteln sehr gut venenverträglich, d.h. sie können unverdünnt intravenös appliziert werden.

Als Wirkstoffe für die erfindungsgemäße Injektionslösung sind das Meglumin- und das Triethanolamin-Azosemid-Salz besonders bevorzugt. Von diesen ist das Azosemid-Salz des Meglumins am günstigsten, da es den niedrigsten Äquivalenzpunkt und überraschenderweise die beste Löslichkeit hat. Bevorzugt wird das organische Lösungsmittel aus der Gruppe Propylenglykol, Polyethylenglykol und Ethanol ausgewählt, wobei Propylenglykol besonders bevorzugt ist.

Es ist günstig, wenn 5 bis 25 Gewichts-% organisches Lösungsmittel in die Rezeptur aufgenommen werden. Die folgende Tabelle zeigt quantitativ, wie durch den Zusatz von organischen Lösungsmitteln, z.B. von Propylenglykol, zu dem Wasser für Injektionszwecke die Löslichkeit des Meglumin- und des Triethanolamin-Azosemid-Salzes wesentlich erhöht werden kann. Zum Vergleich sind in der Tabelle auch entsprechende Werte des Natrium-Azosemidsalzes angegeben.

**Tabelle 1**

| **Kation** | **pH-Wert** | **org. Lösungsmittel-Zusatz in Gew.%** | **Konzentration des Azosemids in mg/ml** |
|---|---|---|---|
| Natrium | 8,0 | - | 1,7 |
| | 10,0 | - | 2,01 |
| | 8,0 | 10 | 3,5 |
| | 10,0 | 10 | 5,5 |
| Meglumin | 9,0 | - | 5,1 |
| | 9,0 | 10 | 11,8 |
| Triethanolamin | 9,8 | 10 | 10,0 |

Bei Verwendung des Meglumin-Azosemid-Salzes läßt sich - wie sich durch Extrapolation aufgrund der in der Tabelle aufgelisteten Werte ergibt - bei einem pH-Wert von 10 und einem Anteil des organischen Lösungsmittels von nahe 25 Gew.% die obere Grenze (20mg/1ml Lösung) der brauchbaren Wirkstoftkonzentration in der erfindungsgemäßen Lösung erreichen. Dabei ist der übliche Sicherheitsabstand zur maximalen Löslichkeit gewahrt. Bei der Entwicklung von Injektionslösungen sollte immer ein Sicherheitsabstand zur Sättigungslöslichkeit eingehalten werden, da das Arzneimittel auch im Winter, d.h. eventuell bei sehr niedrigen Temperaturen, transportiert wird und dabei Ausfällungen des Wirkstoffs auftreten können. Da der ausgefallene Wirkstoff sich dann beim Erwärmen auf Raumtemperatur wieder sehr schnell auflösen muß, sollte der Sicherheitsabstand zur maximalen Löslichkeit mindestens 20 - 30% betragen.

Bevorzugt weisen die Injektionslösungen Titrationsbasizitäten auf die niedriger als 0,3 val/1 ml Injektionslösung (entspr. 0,3 mmol HCl/ml) sind, d.h. der normale Ampulleninhalt von 10 ml Injektionslösung sollte durch Zugabe von höchstens 30 ml 0,1 normale Salzsäure auf den pH-Wert des Blutes von 7,4 gebracht werden können. Die pH-Werte und Titrationsbasizitäten der in der DE 24 23 550 und in dem oben genannten Artikel offenbarten Injektionslösungen (pH: 10,6-11, Titrationsbasizität: >0,3 mmol HCl/ml bzw. pH: 11-12, Titrationsbasizität: 0,4-0,5 mmol HCl/ml) lagen dagegen nicht hoch. 10% Propylenglykol enthaltende, wässrige Lösungen des Natrium-Azosemidsalzes wurden in Tierversuchen getestet. Sie waren schlecht venenverträglich und somit für die Anwendung am Menschen nicht geeignet.

Günstig ist es, wenn in der Lösung eine Pufferkapazität von < 0,1 mmol/ml Injektionslösung eingestellt wird. Es ist vorteilhaft, als Puffer eine Verbindung aus der Gruppe Natriumcarbonat, Arginin-Natrium-Phosphat, N-Methyl-Glucosamin und Trometamol einzusetzen. Es können jedoch prinzipiell auch andere pharmakologisch verträgliche Puffer verwendet werden.

Die ausgehend von einer Suspension des Azosemids in Wasser, gegebenenfalls unter Zusatz eines organischen Lösungsmittels, erfindungsgemäß hergestellten Lösungen weisen, wie die Partikelmessungen zeigen, nach der Sterilisation 0 bis 10 Partikel/ml mit einem Durchmesser von > 10 µm und 0 bis 1 Partikel/ml mit einem Durchmesser von > 25 µm auf, d.h. im Gegensatz zu den aus Lyophilisaten hergestellten Lösungen haben diese spritzfertigen Azosemid-Injektionslösungen eine sehr geringe Partikelzahl.

Zwar läßt sich auch das Ergebnis des Verfahrens, bei dem die Injektionslösung aus einem Lyophilisat hergestellt wird, wesentlich verbessern, wenn statt Wasser erfindungsgemäß ein Wasser/organisches Lösungsmittel-Gemisch als Lösungsmittel eingesetzt wird, weil dann Lösungen hergestellt werden können, die wesentlich höher konzentriert sind, als wenn nur Wasser verwendet wird. Man muß aber nach wie vor die oben erwähnten Nachteile bei der Herstellung über ein Lyophilisat als Zwischenprodukt und die relativ hohe Partikelzahl berücksichtigen.

Weitere vorteilhafte Ausgestaltungen der erfindungsgemäßen Lösung und des erfindungsgemäßen Verfahrens sind in Unteransprüchen offenbart.

Bei dem bevorzugten Verfahren zur Herstellung der erfindungsgemäßen Injektionslösungen wird das mikronisierte Azosemid, Teilchengröße bevorzugt >= 20 µm, in einem aus Wasser für Injektionszwecke und einem organischen Lösungsmittel bestehenden Gemisch suspendiert und durch Umsetzung mit ebenfalls zu dem Gemisch gegebenen Substanzen, wie Meglumin und Triethanolamin, unter Salzbildung gelöst. Der erforderliche pH-Wert wird eingestellt. Wenn erforderlich wird auch noch Puffer zugegeben. Das Meglumin-Azosemid-Salz hat von den genannten Salzen die beste Löslichkeit und den niedrigsten Äquivalenzpunkt (der Aquivalenzpunkt des Megluminsalzes liegt bei pH 8,6). Danach erfolgt die Sterilfiltration, die Abfüllung in Ampullen sowie die Sterilisation der Injektionslösung, die bevorzugt 20 Min. lang bei 121° C durchgeführt wird. Als organische Lösungsmittel werden Propylenglykol, Polyethylenglykol (bevorzugt Polyethylenglykol 400) und Ethanol eingesetzt, von denen das Propylenglykol besonders geeignet ist. Je nach der erforderlichen Wirkstoffkonzentration und dem eingestellten pH-Wert werden 5 bis 25 Gew.% des Lösungsmittels zugefügt. Vorzugsweise werden ca. 20 bis 200 mg/10 ml-Ampulle des Wirkstoffes eingesetzt. Die so hergestellten Lösungen sind mindestens drei Jahre ohne Trübung oder Zersetzung haltbar. Die erfindungsgemäßen Injektionslösungen können auch mit einer 5 %igen Glukose-Infusionslösung oder mit einer isotonischen Kochsalzlösung gemischt und danach infundiert werden. Die erfindungsgemäßen Lösungen sind selbst bei höheren Wirkstoffkonzentrationen stabil, klar und gut venenverträglich. Representative Partikelmessungen ergaben folgende Werte: Partikel mit einem Durchmesser von >= 10µm : 0 - 10 Partikel / ml; Partikel mit einem Durchmesser von >= 25 µm : 0 - 1 Partikel / ml.

Die nachfolgenden Ausführungsbeispiele, bei denen das im vorangegangenen Absatz beschriebene Verfahren angewandt wird, dienen der genaueren Erläuterung der Erfindung und offenbaren die Eigenschaften der erfindungsgemäßen Lösungen.

### Beispiel 1:

### Azosemid 100 mg/10 ml Injektionslösung mit N-Methyl-glukosamin

In einem sterilen, mit Rührwerk versehenen 100 l V2A-Doppelmantelkessel werden 80 l Wasser für Injektionszwecke mit 10 l Propylenglykol gemischt. Darin werden 650 g N-Methylglukosamin gelöst und auf 40 - 50 C° erwärmt. 1 kg Azosemid wird unter Rühren bei 40 - 50 C° gelöst. Danach wird mit Wasser für Injektionszwecke ad 100 l aufgefüllt. Die Lösung hat einen pH-Wert von 8,8 - 9,0. Die Lösung wird über ein Membranfilter der Porenweite 0,2 µm sterilfiltriert. Die sterilfiltrierte Lösung wird zu 10,2 ml in Ampullen abgefüllt. Die gefüllten und verschlossenen Ampullen werden bei 121 C° 20 Min. im Autoklaven sterilisiert.

Man erhält so eine klare, gut verträgliche, spritzfertige Injektionslösung, die mindestens drei Jahre ohne Trübung oder Zersetzung haltbar ist.

Der pH-Wert dieser Lösung beträgt 8,95. Die Titrationsbasizität entspricht 0,006 mmol HCl/ml Injektionslösung, die benötigt werden zum Absenken des pH-Wertes der Injektionslösung von 8,95 auf 7,4 (pH-Wert des Blutes).

### Beispiel 2:

### Azosemid 20 mg/2 ml Injektionslösung mit Triethanolamin

In einem sterilen, mit Rührwerk versehenen 20 l V2A-Doppelmantelkessel werden 15 l Wasser für Injektionszwecke und 2 l Propylenglykol gemischt. In diese Mischung werden 98 g Triethanolamin gelöst und die Lösung auf 40 - 50° C erwärmt. 200 g Azosemid werden unter Rühren hinzugefügt und gelöst und mit Wasser für Injektionszwecke ad 20 l aufgefüllt.

Die Lösung wird über Membranfilter der Porenweite 0,2 µm sterilfiltriert, zu 2,2 ml in Ampullen abgefüllt und die verschlossenen Ampullen im Autoklaven bei 121° C 20 Min. lang sterilisiert.

Der pH-Wert der Lösung beträgt 9,8. Die Titrationsbasizität entspricht 0,05 mmol HCl/ml Injektioslösung, die benötigt werden zum Absenken des pH-Werts von 9,8 auf 7,4.

### Beispiel 3:

### Azosemid 20 mg/2 ml Injektionslösung mit N-Methylglucosamin

In einem sterilen, mit Rührwerk versehenen 100 l V2A- Doppelmantelkessel werden 80 l Wasser für Injektionszwecke mit 10 l Propylenglykol gemischt. Darin werden 526,4 g N-Methylglucosamin und 125g Natriumcarbonat gelöst und auf 40 bis 50° C erwärmt. 1 kg Azosemid wird unter Rühren bei 40 bis 50 C gelöst.

Danach wird mit Wasser für Injektionszwecke ad 100 l aufgefüllt. Die Lösung hat einen pH-Wert von 9,2 bis 9,5. Die Lösung wird über ein Membranfilter der Porenweite 0,2 µm sterilfiltriert. Die sterilfiltrierte Lösung wird zu 2,2 ml in Ampullen abgefüllt. Die gefüllten und verschlossenen Ampullen werden bei 121 °C 20 Min. im Autoklaven sterilisiert.

Man erhält so eine klare, gut verträgliche, spritzfertige Injektionslösung, die mindestens drei Jahre ohne Trübung oder Zersetzung haltbar ist.

Der pH-Wert dieser Lösung beträgt 9,4. Die Titrationsbasizität entspricht 0,009 mmol HCl/ml Injektionslösung, die benötigt werden zum Absenken des pH-Werts der Injektionslösung von 9,4 auf 7,4.

Ein anderes brauchbares Verfahren zum Herstellen der erfindungsgemäßen Injektionslösungen verläuft über die Lyophilisation. Man lyophilisiert den Wirkstoff in alkalischer Lösung und löst das Lyophilisat unter Zuhilfenahme einer Lösungsmittelampulle, in der sich neben Wasser für Injektionszwecke organisches Lösungsmittel und Tensid befinden.

## Patentansprüche

1. Spritzfertige, wässrige Injektionslösung eines wasserlöslichen, physiologisch gut verträglichen Salzes des Azosemids, welche organisches Lösungsmittel enthält, dadurch gekennzeichnet, daß sie mindestens ein Azosemidsalz eines organischen Amins und gegebenenfalls physiologisch gut verträglichen Puffer enthält.

2. Injektionslösung nach Anspruch 1, dadurch gekennzeichnet, daß sie das Meglumin (N-Methyl-Glucosamin)- und/oder das Triethanolamin-Azosemid-Salz enthält.

3. Injektionslösung nach Anspruch 2, dadurch gekennzeichnet, daß das Lösungsmittel aus der Gruppe Propylenglykol, Polyethylenglykol und Ethanol ausgewählt ist und bevorzugt in einer Konzentration von 5 - 25 Gew.% eingesetzt wird.

4. Injektionslösung nach einem der Ansprüche 1 bis 3, dadurch gekennzeichnet, daß der Puffer aus der Gruppe Natriumcarbonat, Arginin-Natrium-Phosphat, N-Methylglucosamin und Trometamol ausgewählt ist.

5. Injektionslösung nach einem der Ansprüche 1 bis 4, dadurch gekennzeichnet, daß ihr pH-Wert > 7 ist.

6. Injektionslösung nach einem der Ansprüche 1 bis 5, dadurch gekennzeichnet, daß die Titrationsbasizität maximal etwa 0,3 mmol HCl/ml Injektionslösung beträgt.

7. Injektionslösung nach Anspruch 5 oder 6, dadurch gekennzeichnet, daß der pH-Wert zwischen 8,9 und 10,3 liegt, daß die Lösung einen physiologisch gut verträglichen, alkalischen Puffer enthält und die Titrationsbasizität zwischen 0,001 und 0,06 mmol HCl/ml Injektionslösung liegt.

8. Verfahren zur Herstellung einer spritzfertigen, wässrigen Injektionslösung mindestens eines wasserlöslichen, physiologisch gut verträglichen Salzes des Azosemids, insbesondere nach einem der Ansprüche 1 bis 7, dadurch gekennzeichnet, daß das Salz in einem Gemisch gelöst wird, welches Wasser, organisches Lösungsmittel und gegebenenfalls physiologisch gut verträglichen Puffer enthält.

9. Verfahren nach Anspruch 8, dadurch gekennzeichnet, daß in einem Wasser und organisches Lösungsmittel enthaltenden Gemisch Salzbildner zum Erzeugen von Azosemidsalzen und gegebenenfalls physiologisch gut verträglicher Puffer gelöst werden und Azosemid suspendiert und unter Salzbildung gelöst wird und der pH-Wert auf einen festgelegten Wert eingestellt wird.

10. Verfahren nach Anspruch 8 oder 9, dadurch gekennzeichnet, daß in der Lösung eine Pufferkapazität von maximal 0,1 mmol HCl/ml Injektionslösung und bevorzugt von maximal 0,03 mmol HCl/ml Injektionslösung eingestellt wird.

11. Verfahren nach einem der Ansprüche 8 bis 10, dadurch gekennzeichnet, daß die Lösung sterilfiltriert, in Ampullen abgefüllt und bevorzugt bei 121 °C 20 Minuten lang sterilisiert wird.

12. Verfahren nach einem der Ansprüche 8 bis 10, dadurch gekennzeichnet, daß der Wirkstoffin alkalischer Lösung lyophilisiert wird und das Lyophilisat in einer Mischung aufgelöst wird, welche neben Wasser für Injektionszwecke organisches Lösungsmittel und Tensid enthält.

## Claims

1. A ready-to-inject aqueous injection solution of a Water-soluble, physiologically well-tolerated salt of azosemide which contains an organic solvent, wherein it contains at least one azosemide salt of an organic amine and optionally a physiologically well-tolerated buffer.

2. Injection solution as claimed in claim 1, wherein it contains the meglumine (N-methyl-glucosamine)azosemide and/or triethanolamine-azosemide salt.

3. Injection solution as claimed in claim 2, wherein the solvent is selected from the group propylene glycol, polyethylene glycol and ethanol and is preferably used at a concentration of 5 to 25 % by weight.

4. Injection solution as claimed in one of the claims 1 to 3, wherein the buffer is selected from the group sodium carbonate, arginine sodium phosphate, N-methylglucosamine and Trometamol.

5. Injection solution as claimed in one of the claims 1 to 4, wherein its pH value is > 7.

6. Injection solution as claimed in one of the claims 1 to 5, wherein the titration basicity is at most about 0.3 mmol HCl/ml injection solution.

7. Injection solution as claimed in claim 5 or 6, wherein the pH value is between 8.9 and 10.3, the solution contains a physiologically well-tolerated, alkaline buffer and the titration basicity is between 0.001 and 0.06 mmol HCl/ml injection solution.

8. Process for the production of a ready-to-inject, aqueous injection solution which contains at least one water-soluble, physiologically well-tolerated salt of azosemide, in particular as claimed in one of the claims 1 to 7, wherein the salt is dissolved in a mixture which contains water, an organic solvent and optionally a physiologically well-tolerated buffer.

9. Process as claimed in claim 8, wherein salt formers for producing azosemide salts and optionally a physiologically well-tolerated buffer are dissolved in a mixture containing water and an organic solvent and azosemide is suspended and dissolved with salt formation and the pH value is adjusted to a defined value.

10. Process as claimed in claim 8 or 9, wherein the buffer capacity in the solution is adjusted to a maximum of 0.1 mmol HCl/ml injection solution and preferably to a maximum of 0.03 mmol HCl/ml injection solution.

11. Process as claimed in one of the claims 8 to 10, wherein the solution is sterile filtered, filled into ampoules and preferably sterilized for 20 minutes at 121°C.

12. Process as claimed in one of the claims 8 to 10, wherein the active substance is lyophilised in an alkaline solution and the lyophilisate is dissolved in a mixture which contains an organic solvent and a surfactant apart from water for injection purposes.

## Revendications

1. Solution injectable aqueuse, prête à l'injection, d'un sel d'azosemide hydrosoluble, physiologiquement acceptable, qui contient un solvant organique, caractérisée en ce qu'elle contient au moins un sel d'azosemide d'une amine organique et le cas échéant, un tampon physiologiquement acceptable.

2. Solution injectable selon la revendication 1, caractérisée en ce qu'elle contient le sel de méglumine (N-méthyl-glucosamine) et/ou le sel de triéthanolamine de l'azosemide.

3. Solution injectable selon la revendication 2, caractérisée en ce que le solvant est choisi dans le groupe formé par le propylèneglycol, le polyéthylèneglycol et l'éthanol et il est mis en oeuvre en une concentration de 25 % en poids.

4. Solution injectable selon l'une quelconque des revendications 1 à 3, caractérisée en ce que le tampon est choisi dans le groupe formé par le carbonate de sodium, le phosphate d'arginine sodique, la N-méthylglucosamine et le trométamol.

5. Solution injectable selon l'une quelconque des revendications 1 à 4, caractérisée en ce que son pH est de 7.

6. Solution injectable selon l'une quelconque des revendications 1 à 5, caractérisée en ce que sa basicité à la titration est au maximum d'environ 0,3 mmole de HCl/ml de solution injectable.

7. Solution injectable selon la revendication 5 ou 6, caractérisée en ce que son pH est compris entre 8,9 et 10,3, que la solution contient un tampon alcalin physiologiquement acceptable et que sa basicité à la titration est comprise entre 0,001 et 0,06 mmole de HCl/ml de solution injectable.

8. Procédé de préparation d'une solution injectable aqueuse, prête à l'injection, d'au moins un sel d'azosemide hydrosoluble, physiologiquement acceptable, en particulier selon l'une quelconque des revendications 1 à 7, caractérisé par le fait que le sel est dissous dans un mélange qui contient de l'eau, un solvant organique et le cas échéant, un tampon physiologiquement acceptable.

9. Procédé selon la revendication 8, caractérisé en ce que, dans un mélange contenant de l'eau et un solvant organique, on dissout des formateurs de sel pour obtenir des sels d'azosemide et le cas échéant, un tampon physiologiquement acceptable et on met en suspension de l'azosemide et le dissout par formation de sels, et on ajuste le pH à une valeur prédéterminée.

10. Procédé selon la revendication 8 ou 9, caractérisé en ce que dans la solution, on ajuste une capacité de tampon de 0,1 mmole de HCl/ml de solution injectable au maximum, et de préférence, de 0,03 mmole de HCl/ml de solution injectable au maximum.

11. Procédé selon l'une quelconque des revendications 8 à 10, caractérisé en ce que l'on filtre la solution dans des conditions stériles, on la conditionne dans des ampoules et on la stérilise de préférence à 121°C pendant 20 minutes.

12. Procédé selon l'une quelconque des revendications 8 à 10, caractérisé en ce que le principe actif est lyophilisé en solution alcaline et que le lyophilisat est redissout dans un mélange qui contient, en plus d'eau pour solution injectable, un solvant organique et un tensioactif.
